# EUROPEAN PATENT APPLICATION

(11) **EP 4 495 221 A1**
(43) Date of publication of application: **22.01.2025**
(21) Application number: 23769623.2
(22) Date of filing: 08.03.2023
(51) Int. Cl.: C12M 1/38, C12M 1/02, C12M 1/00

(54) **SYSTEM AND METHOD FOR TREATING BIOLOGICAL SAMPLE**

(30) Priority: 17.03.2022 CN 202210262700; 29.06.2022 CN 202210747882
(71) Applicant: Suzhou Abogen Biosciences Co., Ltd., Suzhou, Jiangsu 215123 (CN)
(72) Inventor: YAN, Huayuan, Suzhou, Jiangsu 215123 (CN); FAN, Xiangchen, Suzhou, Jiangsu 215123 (CN)
(74) Representative: Epping - Hermann - Fischer
(86) International application number: PCT/CN2023/080232
(87) International publication number: WO 2023/174117

(57) **Abstract**

The present application relates to the technical field of biological sample treatment, and provides a system for treating a biological sample. The system comprises: a temperature regulation assembly used for changing temperature of a biological sample flowing therethrough; and a reactor array coupled to the temperature regulation assembly. The reactor array comprises two or more reactors, and each reactor is configured to cause the biological sample to undergo at least one treatment. The treatment is performed in two or more reactors alternately, such that a time interval between completion of discharge of the treated biological sample from a first reactor and start of discharge of the treated biological sample from a second reactor immediately next to the first reactor is less than a predetermined time period. The present application also provides a method for treating a biological sample. According to the system of the present application, the time of each reaction is shortened, so that the waiting time before each discharge of a reacted biological sample is reduced. In some cases, the reactor array, taken as a whole, can output treated biological samples without interruption and continuously.

## Description

### TECHNICAL FIELD

The present disclosure relates to systems and methods for treating biological samples, and more particularly to a treatment system including a plurality of reactors and a control method thereof.

### BACKGROUND

The reaction system used in laboratory research has different requirements for parameters such as reactor capacity, reaction time and reaction efficiency from the reaction system used in industrial production. For example, in small-scale laboratory studies, 1.5 ml centrifuge tubes can be used for pre-denaturation before mRNA capping, PCR (polymerase chain reaction), or reverse transcription reactions of nucleic acid (e.g., DNA or RNA), thereby achieving rapid heating (e.g., using a metal bath or a water bath, which can achieve heating to a predetermined temperature within 5 min) and cooling (e.g., placing the centrifuge tube in an ice or low-temperature environment, which can achieve cooling to a predetermined temperature within 5 min). However, since the speed of heating and cooling decreases significantly with the increase of the reactor capacity, it will be difficult to achieve the above-mentioned rapid heating and cooling effects on a laboratory scale as the reactor capacity increases. For example, an existing temperature swing reactor system that uses a TCU-jacket/heating blanket to control temperature typically requires 30 min to achieve temperature increase or decrease. This will have an adverse effect on the reaction system. For example, if the cooling time is too long, DNA or RNA may reform secondary structures, affecting the reaction efficiency.

### SUMMARY OF THE INVENTION

In one aspect, the present disclosure provides a system for treating a biological sample, the system comprising: a temperature regulation assembly configured to change the temperature of a biological sample flowing therethrough; and a reactor array coupled to the temperature regulation assembly, the reactor array comprising two or more reactors, each reactor in the reactor array being configured to cause the biological sample to undergo at least one treatment, each of the reactors having an input port configured to receive the biological sample and an output port configured to discharge the treated biological sample, wherein the treatment is performed in the two or more reactors alternately, such that the time interval between completion of discharge of the treated biological sample from a first reactor and start of discharge of the treated biological sample from a second reactor is less than a predetermined time period, the second reactor being located immediately next to the first reactor in the reactor array.

In another aspect, the present disclosure provides a method for treating a biological sample, the method comprising: changing the temperature of a biological sample to a first predetermined temperature using a temperature regulation assembly; allowing the biological sample to flow from the temperature regulation assembly to a reactor array, the reactor array comprising two or more reactors, each of the reactors having an input port configured to receive the biological sample and an output port configured to discharge the treated biological sample; causing the biological sample to undergo at least one treatment in at least one reactor in the reactor array; and discharging the treated biological sample from the reactor array to the temperature regulation assembly, and changing the temperature of the treated biological sample to a second predetermined temperature using the temperature regulation assembly, wherein the treatment is performed in the two or more reactors alternately, such that the time interval between completion of discharge of the treated biological sample from a first reactor and start of discharge of the treated biological sample from a second reactor is less than a predetermined time period, the second reactor being immediately next to the first reactor in the reactor array.

In another aspect, the present disclosure provides a system for treating a biological sample, the system comprising: a temperature regulation assembly configured to change the temperature of a biological sample flowing therethrough; and a reactor array coupled to the temperature regulation assembly, the reactor array comprising two or more reactors, each reactor in the reactor array being configured to cause the biological sample to undergo at least one treatment, each of the reactors having an input port configured to receive the biological sample and an output port configured to discharge the treated biological sample, wherein the treatment in the two or more reactors is coordinated such that the time interval between completion of the treatment in a first reactor and completion of the treatment in a second reactor is less than a predetermined time period, the second reactor being immediately next to the first reactor in the reactor array.

In another aspect, the present disclosure provides a method for treating a biological sample, the method comprising: changing the temperature of a biological sample to a first predetermined temperature using a temperature regulation assembly; allowing the biological sample to flow from the temperature regulation assembly to a reactor array, the reactor array comprising two or more reactors, each of the reactors having an input port configured to receive the biological sample and an output port configured to discharge the treated biological sample; causing the biological sample to undergo at least one treatment in at least one reactor in the reactor array; and discharging the treated biological sample from the reactor array to the temperature regulation assembly, and changing the temperature of the treated biological sample to a second predetermined temperature using the temperature regulation assembly, wherein the treatment in the two or more reactors is coordinated such that the time interval between completion of the treatment in a first reactor and completion of the treatment in a second reactor is less than a predetermined time period, the second reactor being immediately next to the first reactor in the reactor array.

In another aspect, the present disclosure provides a system for treating a biological sample, the system comprising: a temperature regulation assembly configured to change the temperature of a biological sample flowing through it; and a reactor array coupled to the temperature regulation assembly, the reactor array comprising two or more reactors, each of the reactors having an input port configured to receive the biological sample and an output port configured to discharge the treated biological sample, wherein the treatment is performed in the two or more reactors alternately, and each reactor in the reactor array is configured to cause the biological sample to undergo at least one treatment within a time period not exceeding a predetermined time period.

In another aspect, the present disclosure provides a method for treating a biological sample, the method comprising: changing the temperature of a biological sample to a first predetermined temperature using a temperature regulation assembly; allowing the biological sample to flow from the temperature regulation assembly to a reactor array, the reactor array comprising two or more reactors, each of the reactors having an input port configured to receive the biological sample and an output port configured to discharge the treated biological sample; causing the biological sample to undergo at least one treatment in the two or more reactors alternately, wherein the biological sample undergoes the at least one treatment in the at least one reactor within a time period not exceeding a predetermined time period; and discharging the treated biological sample from the reactor array to the temperature regulation assembly, and changing the temperature of the treated biological sample to a second predetermined temperature using the temperature regulation assembly.

Other aspects and advantages of the present disclosure will become apparent to those skilled in the art from the following detailed description, wherein only illustrative embodiments of the present disclosure are shown and described. As will be realized, the present disclosure can include other and different embodiments, and the details thereof can be modified in various obvious respects, all of which do not depart from the present disclosure. Accordingly, the drawings and description are to be regarded as illustrative in nature, and not restrictive.

### DESCRIPTION OF DRAWINGS

The novel features of the present disclosure are set forth with particularity in the appended claims. A better understanding of the features and advantages of the present disclosure will be obtained by referring to the following detailed description and accompanying drawings which set forth illustrative embodiments in which the principles of the present disclosure are utilized, in which:
FIG. 1 is a schematic diagram depicting a system for treating a biological sample according to the present disclosure.
FIG. 2 is a schematic diagram depicting an exemplary embodiment of a system for treating a biological sample according to the present disclosure.
FIG. 3A is a schematic diagram depicting another exemplary embodiment of a system for treating a biological sample according to the present disclosure.
FIG. 3B is a schematic diagram depicting yet another exemplary embodiment of a system for treating a biological sample according to the present disclosure.
FIG. 4 is a flow chart depicting an exemplary method for treating a biological sample according to the present disclosure.
FIG. 5 shows a computer system programmed or otherwise configured to implement the method for treating a biological sample provided by the present disclosure.

### DETAILED DESCRIPTION

Although various embodiments of the present disclosure have been shown and described herein, it will be apparent to those skilled in the art that these embodiments are provided in an exemplary way only. Numerous variations, changes, and substitutions may occur to those skilled in the art without departing from the present disclosure. It should be understood that various alternatives to the embodiments of the present disclosure described herein may be employed.

As used in the Description and the Claims, the singular forms "a," "an" and "the" include plural referents, unless the context otherwise expressly states. For example, the term "a cell" includes a plurality of cells, including a mixture thereof.

As used herein, the term "amplification" generally refers to the generation of one or more copies or "amplification products" of nucleic acid. The term "DNA amplification" generally refers to the generation of one or more copies of a DNA molecule or "amplified DNA product". The term "reverse transcription amplification" generally refers to the production of deoxyribonucleic acid (DNA) from a ribonucleic acid (RNA) template by reverse transcriptase.

As used herein, the term "denaturation" generally refers to the complete or partial unwinding of the helical structure of double-stranded nucleic acid and, in some cases, to the unwinding of the secondary structure of single-stranded nucleic acid. Denaturation can include inactivation of pathogen cell walls or viral coats, as well as inactivation of inhibitor proteins. The conditions under which the denaturation can occur include a "denaturation temperature", which generally refers to the temperature at which the denaturation is permitted, and a "denaturation duration", which generally refers to the amount of time allocated for the denaturation to occur.

As used herein, the term "extension" generally refers to the incorporation of nucleotides into a nucleic acid as directed by a template. Extension may occur with the aid of an enzyme, such as a polymerase or a reverse transcriptase. The conditions under which extension can occur include "extension temperature" which generally refers to the temperature that allows extension to occur, and "extension duration" which generally refers to the amount of time allotted for extension to occur.

As used herein, the term "nucleic acid" generally refers to a polymeric form of nucleotides of any length (deoxyribonucleotides (dNTPs) or ribonucleotides (rNTPs)) or their analogs. Nucleic acid can have any three-dimensional structure and can perform any function, known or unknown. Non-limiting examples of nucleic acid include DNA, RNA, coding or noncoding regions of a gene or gene fragment, one or more loci determined by linkage analysis, exons, introns, messenger RNA (mRNA), transfer RNA, ribosomal RNA, short interfering RNA (siRNA), short hairpin RNA (shRNA), micro RNA (miRNA), ribozymes, cDNA, recombinant nucleic acids, branched nucleic acids, plasmids, vectors, isolated DNA of any sequence, isolated RNA of any sequence, nucleic acid probes, and primers. Nucleic acid may contain one or more modified nucleotides, such as methylated nucleotides and nucleotide analogs. Modification to the nucleotide structure, if any, may be made before or after nucleic acid assembly. The nucleotide sequence of nucleic acid may be interrupted by non-nucleotide components. Nucleic acid can be further modified after polymerization, for example by coupling or conjugation to a reporter.

As used herein, the term "primer extension reaction" generally refers to the denaturation of double-stranded nucleic acid, the binding of primers to one or both strands of the denatured nucleic acid, and subsequent extension of the primers.

As used herein, the term "target nucleic acid" generally refers to a nucleic acid molecule with a certain nucleotide sequence in a starting population of nucleic acid molecules, and the presence, amount and/or sequence of which or a change in one or more of them needs to be determined. The target nucleic acid can be any type of nucleic acid, including DNA, RNA, and their analogs. As used herein, "target ribonucleic acid (RNA)" generally refers to the target nucleic acid as the RNA. As used herein, "target deoxyribonucleic acid (DNA)" generally refers to the target nucleic acid as the DNA.

As used herein, the term "biological sample" generally refers to any sample taken from the subject's target position. Non-limiting examples of biological samples include blood (or ingredients of blood - e.g., white blood cells, red blood cells, platelets) obtained from any anatomical position of a subject (e.g., tissue, circulatory system, bone marrow), cells obtained from any anatomical position of a subject, skin, heart, lung, kidney, exhaled breath, bone marrow, stool, semen, vaginal fluid, tissue fluid from tumor tissue, breast, pancreas, cerebrospinal fluid, tissue, throat swab, bioptic target, placental fluid, amniotic fluid, liver, muscle, smooth muscle, bladder, gall bladder, colon, intestine, brain, coelomic fluid, sputum, pus, micropiota, meconium, milk, prostate, esophagus, thyroid, serum, saliva, urine, gastric and digestive fluid, tear, ocular fluid, sweat, mucus, earwax, oil, glandular secretions, cerebrospinal fluid, hair, nails, skin cells, plasma, nasal swab or nasopharyngeal wash, cerebrospinal fluid, umbilical cord blood, and/or other excreta or body tissues. Non-limiting examples of biological samples also include proteins and nucleic acid molecules synthesized in vivo or in vitro. Non-limiting examples of biological samples also include artificially synthesized nucleic acid molecules.

As used herein, the term "reactor" generally refers to a device in which reactants undergo a chemical or biological reaction in production or experiments. The reactor can be characterized by various condition parameters in the reaction process, such as pressure, temperature, volume, residence time, concentration of each reactant, reaction rate, heat transfer coefficient, stirring rate. Examples of reactors may include, but are not limited to, fermenters, incubation tanks, and the like.

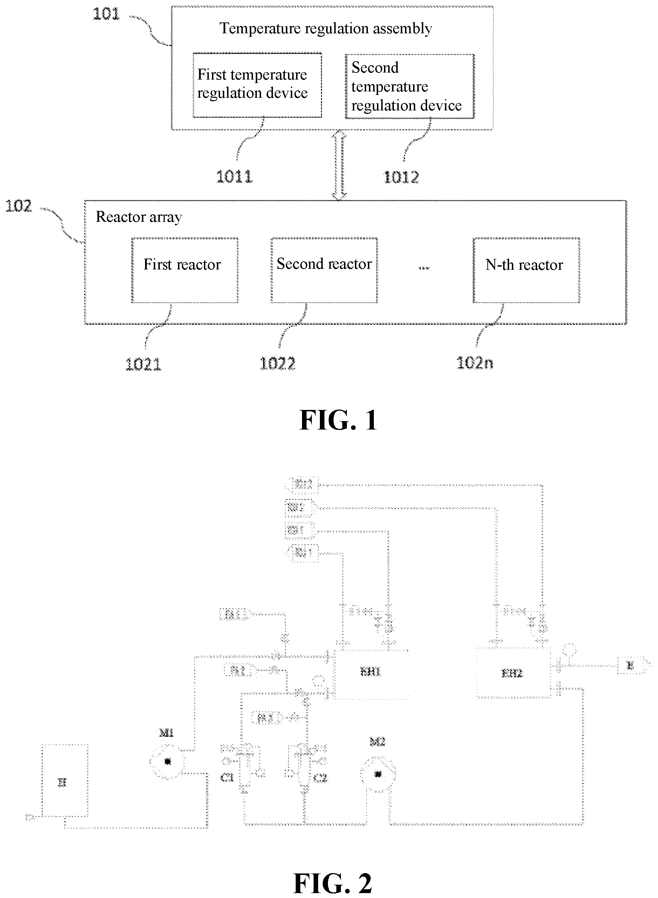

FIG. 1 is a schematic diagram depicting a system for treating a biological sample according to the present disclosure. In some exemplary embodiments, the system for treating a biological sample may include a temperature regulation assembly 101 and a reactor array 102 coupled to the temperature regulation assembly 101. The temperature regulation assembly is configured to change the temperature of a biological sample flowing therethrough. A temperature regulation assembly may be provided for each reactor in the reactor array separately, or a temperature regulation assembly may be provided for a plurality of reactors in the reactor array. The temperature regulation assembly can receive a biological sample (e.g., a liquid material with nucleic acid; the nucleic acid can include messenger ribonucleic acid (mRNA)) from a sample source (e.g., a feeder), and perform a first temperature regulation treatment on the biological sample. The temperature regulation assembly can also receive the biological sample after the predetermined treatment from the reactor array, and perform a second temperature regulation treatment on the biological sample after the predetermined treatment. In an example, the first temperature regulation treatment includes a heating treatment, and the second temperature regulation treatment includes a cooling treatment. The reactor array can be in fluid communication with the temperature regulation assembly and receive the biological sample having undergone at least one temperature regulation treatment from the temperature regulation assembly. Each reactor in the reactor array can cause the biological sample to undergo at least one treatment (e.g., nucleic acid denaturation) therein. Optionally, the biological sample after reaction in the reactor can be returned to the temperature regulation assembly for further temperature regulation. In an example, at least one reactor in the reactor array comprises an incubator.

In some embodiments, the temperature regulation assembly may include a first temperature regulation device 1011 for heating the biological sample and a second temperature regulation device 1012 for cooling the biological sample. As described above, for each reactor in the reactor array, a separate first temperature regulation device and a separate second temperature regulation device may be provided. Alternatively, a common first temperature regulation device and a common second temperature regulation device may be provided for a plurality of reactors in the reactor array. In an example, the first temperature regulation device is configured to increase the temperature of the biological sample to a first predetermined temperature within a first time period. The first time period can be 10 s, 20 s, 30 s, 40 s, 50 s, 60 s, 1 min, 1.5 min, 2 min, 2.5 min, 3 min, 3.5 min, 4 min, 4.5 min, 5 min, 5.5 min, 6 min, 6.5 min, 7 min, 7.5 min, 8 min, 8.5 min, 9 min, 9.5 min, 10 min, 10.5 min, 11 min, 11.5 min, 12 min, 12.5 min, 13 min, 13.5 min, 14 min, 14.5 min, 15 min, 16 min, 17 min, 18 min, 19 min, 20 min, 21 min, 22 min, 23 min, 24 min, 25 min, 26 min, 27 min, 28 min, 29 min or 30 min. The first time period may be a value between any two time periods listed above. In an example, the first time period does not exceed 5 min. The first predetermined temperature may be at least 5°C, 10°C, 15°C, 20°C, 25°C, 30°C, 35°C, 40°C, 45°C, 50°C, 55°C, 60°C, 65°C, 70°C, 75°C, 80°C, 85°C or 90°C. The first predetermined temperature may be a value between any two of the temperatures listed above. In an example, the first temperature regulation device can increase the temperature of the biological sample from room temperature to at least 65°C within 5 min. In some cases, the first temperature regulation device can heat the biological sample in a staged heating manner. For example, the first temperature regulation device may include a plurality of heating modules arranged in series, and each heating module is configured to heat the biological sample individually. Each heating module can be configured to increase the temperature of the biological sample from an initial temperature by a predetermined temperature difference within a predetermined time period.

In an example, the second temperature regulation device is configured to decrease the temperature of the biological sample to a second predetermined temperature within a second time period. The second time period can be 10 s, 20 s, 30 s, 40 s, 50 s, 60 s, 1 min, 1.5 min, 2 min, 2.5 min, 3 min, 3.5 min, 4 min, 4.5 min, 5 min, 5.5 min, 6 min, 6.5 min, 7 min, 7.5 min, 8 min, 8.5 min, 9 min, 9.5 min, 10 min, 10.5 min, 11 min, 11.5 min, 12 min, 12.5 min, 13 min, 13.5 min, 14 min, 14.5 min, 15 min, 16 min, 17 min, 18 min, 19 min, 20 min, 21 min, 22 min, 23 min, 24 min, 25 min, 26 min, 27 min, 28 min, 29 min or 30 min. The second time period may be a value between any two time periods listed above. In an example, the second time period does not exceed 5 min. The second predetermined temperature may be at least 1°C, 5°C, 10°C, 15°C, 20°C, 25°C, 30°C, 35°C, 40°C, 45°C, 50°C, 55°C, 60°C, 65°C, 70°C, 75°C, 80°C, 85°C or 90°C. The second predetermined temperature may be a value between any two of the temperatures listed above. In an example, the second temperature regulation device can decrease the temperature of the biological sample from at least 65°C to 2-8°C within 5 min. In some cases, the second temperature regulation device can cool the biological sample in a staged cooling manner. For example, the second temperature regulation device may include a plurality of cooling modules arranged in series, and each cooling module is configured to cool the biological sample individually. Each cooling module can be configured to decrease the temperature of the biological sample from an initial temperature by a predetermined temperature difference within a predetermined time period.

In some embodiments, the first temperature regulation device may include a tubular heat exchanger, such as a heating capillary heat exchanger. The heat transfer medium in a liquid state is heated, and the heated heat transfer medium flows through the capillary tube of the first temperature regulation device. The biological sample is heated when it comes into contact with the wall of the capillary tube. In some embodiments, the second temperature regulation device may include a tubular heat exchanger, such as a cooling capillary heat exchanger. The heat transfer medium in a liquid state is cooled, and the cooled heat transfer medium flows through the capillary tube of the second temperature regulation device. The biological sample is cooled when it comes into contact with the wall of the capillary tube. In other embodiments, the first temperature regulation device may include a heating device such as a resistance-type heater (for example, using Joule heat generated by current passing through a conductor as a heat source), a radiant heater (for example, using infrared radiation as a heat source), or a combination thereof, and the second temperature regulation device may include a thermoelectric cooler (for example, utilizing the Peltier effect), a heat sink (for example, metal fins), a shell-and-tube cooler, a plate cooler, and an air-cooled cooler, or a combination thereof.

For the temperature regulation assembly, a temperature control unit (TCU) can be provided to control its operation, which is used to control at least one operating parameter of the first temperature regulation device and the second temperature regulation device (for example, the flow rate of the heat transfer medium, the operating mode), so as to control the above-mentioned first time period, the first predetermined temperature, the second time period, the second predetermined temperature and other parameters. For example, in some cases, the first temperature regulation device or the second temperature regulation device may continuously perform the heating or cooling treatment; in other cases, the first temperature regulation device or the second temperature regulation device may intermittently perform the heating or cooling treatment. In some embodiments, the temperature control unit may include a first temperature control unit for controlling the operation of the first temperature regulation device, and a second temperature control unit for controlling the operation of the second temperature regulation device. In this way, the first temperature regulation device and the second temperature regulation device can be controlled quickly and accurately respectively. The temperature regulation assembly may include a human-computer interaction interface, such as a control panel or a software interface. The operator of the biological sample treatment system according to the present disclosure can set, view and modify at least one operating parameter of the temperature regulation assembly through the human-machine interaction interface.

In some embodiments, the reactor array 102 may include two or more reactors, such as a first reactor 1021, a second reactor 1022, and optionally an N-th reactor 102n. The two or more reactors in the reactor array may have the same capacity. The two or more reactors in the reactor array may have different capacities. The capacity of at least one reactor in the reactor array may be no more than 100 L, 90 L, 80 L, 70 L, 60 L, 50 L, 40 L, 30 L, 25 L, 20 L, 15 L, 10 L, 9.5 L, 9.0 L, 8.5 L, 8.0 L, 7.5 L, 7.0 L, 6.5 L, 6.0 L, 5.5 L, 5.0 L, 4.5 L, 4.0 L, 3.5 L, 3.0 L, 2.9 L, 2.8 L, 2.7 L, 2.6 L, 2.5 L, 2.4 L, 2.3 L, 2.2 L, 2.1 L, 2.0 L, 1.9 L, 1.8 L, 1.7 L, 1.6 L, 1.5 L, 1.4 L, 1.3 L, 1.2 L, 1.1 L, 1.0 L, 0.9 L, 0.8 L, 0.7 L, 0.6 L, 0.5 L, 0.4 L, 0.3 L, 0.2 L, 0.1 L, 0.09 L, 0.08 L, 0.07 L, 0.06 L, 0.05 L, 0.04 L, 0.03 L, 0.02 L or 0.01 L. The capacity of at least one reactor in the reactor array may be a value between any two of the capacities listed above. In an example, the reactor may have a capacity of 1.5 L. These reactors can be connected in parallel with each other and are in fluid communication with the temperature regulation assembly through a common pipeline or separate pipelines, so that the biological sample can be transferred from the temperature regulation assembly to at least one reactor in the reactor array and/or from at least one reactor in the reactor array to the temperature regulation assembly. These reactors can independently receive and discharge biological samples. In an example, a reactor in a reactor array has an input port configured to receive a biological sample and an output port configured to discharge a treated biological sample. In another example, the input port and the output port may be the same port, which is used as an input port in a first time period and as an output port in a second time period through time division multiplex access.

The transfer of the biological sample between the temperature regulation assembly and at least one reactor in the reactor array can be achieved by a pumping system. The pumping system is configured to perform at least one of the following operations: (i) transfer the biological sample from the biological sample source to the temperature regulation assembly, (ii) transfer the biological sample from the temperature regulation assembly to at least one reactor in the reactor array, and (iii) transfer the biological sample from at least one reactor in the reactor array to the temperature regulation assembly. The pumping system may include at least one pump. Examples of pumps include, but are not limited to, centrifugal pumps, diaphragm pumps, piston pumps, peristaltic pumps, gear pumps, and the like. The pumping system can transfer biological samples at fixed or adjustable flow rates. The pumping rate of at least one pump in the pumping system (e.g., the volume of liquid discharged by the pump per unit time) can be at least 0.001 L/s, 0.002 L/s, 0.003 L/s, 0.004 L/s, 0.005 L/s, 0.006 L/s, 0.007 L/s, 0.008 L/s, 0.009 L/s, 0.01 L/s, 0.02 L/s, 0.03 L/s, 0.04 L/s, 0.05 L/s, 0.06 L/s, 0.07 L/s, 0.08 L/s, 0.09 L/s, 0.1 L/s, 0.2 L/s, 0.3 L/s, 0.4 L/s, 0.5 L/s, 0.6 L/s, 0.7 L/s, 0.8 L/s, 0.9 L/s, or 1 L/s. The pumping rate of at least one pump in the pumping system may be a value between any two of the flow rates listed above. The pumping system is configured to: (i) transfer the biological sample from the biological sample source to the temperature regulation assembly, (ii) transfer the biological sample from the temperature regulation assembly to at least one reactor in the reactor array, and (iii) transfer the biological sample from at least one reactor in the reactor array to the temperature regulation assembly within a predetermined or adjustable time period. The time period can be up to 30 min, 25 min, 20 min, 15 min, 14.5 min, 14 min, 13.5 min, 13 min, 12.5 min, 12 min, 11.5 min, 11 min, 10.5 min, 10 min, 9.5 min, 9 min, 8.5 min, 8 min, 7.5 min, 7 min, 6.5 min, 6 min, 5.5 min, 5 min, 4.5 min, 4 min, 3.5 min, 3 min, 2.5 min, 2 min, 1.5 min, 1 min, 30 s, 10 s or 1 s. The time period may be a value between any two of the time periods listed above.

The reactors in the reactor array are configured to cause the biological sample therein to undergo at least one treatment. In some embodiments, the at least one treatment may include nucleic acid denaturation. The biological sample treated by the system for treating a biological sample disclosed herein can be provided to a thermal cycler (e.g., a PCR thermal cycler) to amplify and modify the nucleic acid of interest in the sample and detect the product of amplification or modification. For example, the biological sample treated by the system for treating a biological sample of the present disclosure can be heated up and cooled down by a thermal cycler, thereby performing a polymerase chain reaction on the reaction mixture containing the biological sample in the container. In other embodiments, the at least one treatment may include nucleic acid amplification. For example, the target nucleic acid can be amplified in at least one reactor of the reactor array to generate a product of amplification. The target nucleic acid can be target RNA or target DNA. In some embodiments, the target RNA is viral RNA. In some embodiments, the target DNA is viral DNA.

In some embodiments, at least one reactor in the reactor array is configured to cause the biological sample to undergo at least one treatment within a predetermined treatment time period. The present treatment time period can be no more than 100 min, 90 min, 80 min, 70 min, 60 min, 50 min, 40 min, 30 min, 29 min, 28 min, 27 min, 26 min, 25 min, 24 min, 23 min, 22 min, 21 min, 20 min, 19 min, 18 min, 17 min, 16 min, 15 min, 14 min, 13 min, 12 min, 11 min, 10 min, 9 min, 8 min, 7 min, 6 min, 5 min, 4 min, 3 min, 2 min, 1 min, 50 s, 40 s, 30 s, 20 s, 10 s or 1 s. The time period may not exceed a value between any two of the time periods listed above. In an example, at least one reactor in the reactor array is configured such that the biological sample completes at least one treatment within a treatment time period of no more than 5 min.

In some embodiments, at least one reactor in the reactor array is configured to process the biological sample at a predetermined treatment temperature. In some cases, the predetermined treatment temperature may be the same as the temperature of the biological sample discharged from the temperature regulation assembly after the temperature regulation treatment. In other cases, the predetermined treatment temperature may be different from the temperature of the biological sample discharged from the temperature regulation assembly after the temperature regulation treatment. A temperature adjustment device may be provided for at least one reactor in the reactor array to adjust the treatment temperature of the biological sample in the reactor. For example, a heater may be provided at the reactor, which is configured to heat, keep warm, or cool the biological sample in the reactor, thereby maintaining the predetermined reaction temperature in the reactor. In an example, the heater can include a resistance-type heater, a radiant heater, or a combination thereof.

In some embodiments, the system for treating a biological sample according to the present disclosure further includes a sensing system coupled to the reactor array. The sensing system is configured to sense at least one parameter of at least one reactor in the reactor array. The sensing system may include at least a thermometer configured to measure the temperature of the biological sample within at least one reactor in the reactor array. The sensing system may include at least a pressure meter configured to measure the pressure within at least one reactor in the reactor array. The sensing system may include at least a weighing device configured to measure the weight of the biological sample within at least one reactor in the reactor array. The sensing system may also include at least a pH value measuring device, a turbidity measuring device and other sensors. At least one parameter of at least one reactor in the reactor array measured by the sensing system can be transmitted to the controller of the system for treating a biological sample according to the present disclosure in real time and compared with a set threshold value, so as to monitor and control the reaction in the reactor. In some embodiments, the system for treating a biological sample according to the present disclosure further includes a waste liquid container coupled to the reactor array. The waste liquid container is used to store waste liquid from the reactors in the reactor array, for example, a flushing liquid after flushing the reactors.

In some embodiments, the treatments in the two or more reactors in the reactor array are coordinated and controlled so that the time interval (t2-t1) between the time t1 when discharge of the biological sample having undergone a predetermined treatment from the first reactor 1021 is completed and the time t2 when discharge of the biological sample having undergone a predetermined treatment from the second reactor 1022 starts is less than a predetermined time period, and the second reactor is immediately next to the first reactor in the reactor array. The predetermined time period may be less than 15 min, 14 min, 13 min, 12 min, 11 min, 10 min, 9 min, 8 min, 7 min, 6 min, 5 min, 4.5 min, 4 min, 3.5 min, 3 min, 2.5 min, 2 min, 1.5 min, 1 min, 55 s, 50 s, 45 s, 40 s, 35 s, 30 s, 25 s, 20 s, 15s, 10 s, 9 s, 8 s, 7 s, 6 s, 5 s, 4 s, 3 s, 2 s or 1 s. The predetermined time period may be less than a value between any two of the time periods listed above. "The time interval between the time when discharge of the biological sample having undergone a predetermined treatment from the first reactor is completed and the time when discharge of the biological sample having undergone a predetermined treatment from the second reactor immediately next to the first reactor starts is less than a predetermined time period" described in the present disclosure can be achieved by coordinated control of at least one of the following parameters: the capacity of the reactors in the reactor array, the time required to complete a predetermined reaction in the reactor (e.g., by controlling reaction environment parameters of the reactor, such as temperature, pressure), the time required to completely transfer the biological sample to the reactors (e.g., the time required to completely transfer a predetermined amount of the biological sample from the temperature regulation assembly to a reactor, which may depend on the pumping rate of the pumping system), the time required to completely transfer the biological sample from the reactors to other components (e.g., the time required to completely transfer the biological sample having undergone a predetermined reaction out from a reactor, which may depend on the pumping rate of the pumping system), the time required to perform other treatments in the reactor (e.g., the time required to rinse the interior of the reactor after the completion of each reaction), etc.

In an example, as shown in FIG. 2 , the reactor array in the system for treating a biological sample according to the present disclosure may include two reactors C1 and C2. The biological sample to be treated is transferred by a pumping system (e.g., M1 in the pumping system) from a biological sample source H via a first temperature regulation device EH1 in the temperature regulation assembly to the two reactors respectively (hereinafter also referred to as "input"), and undergoes a predetermined reaction (hereinafter also referred to as "reaction", e.g., incubation) in the two reactors. After the reaction is completed, the biological sample is transferred from the two reactors by a pumping system (e.g., M2 in the pumping system) to a biological sample collector E via a second temperature regulation device EH2 in the temperature regulation assembly, respectively (hereinafter also referred to as "output"). The first temperature regulation device EH1 may be, for example, a heating device, for which a temperature controller TCU1 may be provided. The second temperature regulation device EH2 may be a cooling device, for example. A temperature controller TCU 2 may be provided for the cooling device.

In an embodiment, the treatment timing sequence of the input, reaction and output operations performed on the two reactors can be as shown in Table 1.

**Table 1**

| Time period | First reactor | Second reactor |
|---|---|---|
| T0 | Input | |
| T1 | Reaction | Input |
| T2 | Output + input | Reaction |
| T3 | Reaction | Output + input |
| T4 | Output + input | Reaction |
| T5 | Reaction | Output + input |
| T6 | Output + input | Reaction |
| ... | ... | ... |
| | | |

In an example, the time periods T0, T1, T2, ... may each be, for example, 5 min. From the timing sequence of operations in Table 1, it is known that the first reactor and the second reactor repeat "output + input" and "reaction" in adjacent timing sequences, respectively, in addition to the two initial time periods of T0 and T1. The time required to complete the predetermined reaction (e.g., incubation) in the first reactor/second reactor is 5 min. In some cases, the pumping system can be so configured that the time required to completely move the biological sample out of the first reactor/second reactor after the incubation is 2.5 min, and the time required to completely move the biological sample to be incubated into the first reactor/second reactor is 2.5 min. In this way, the incubation is completed in the first reactor and the second reactor alternately, and the time interval between the time when discharge of the incubated biological sample from the first reactor is completed and the time when discharge of the incubated biological sample from the second reactor starts is 2.5 min. In other words, in this case, the reactor array, taken as a whole, outputs the incubated biological sample for 2.5 min every 2.5 min. In other cases, the pumping system can be so configured that the time required to completely move the biological sample out of the first reactor/second reactor after the incubation is 3 min, and the time required to completely move the biological sample to be incubated into the first reactor/second reactor is 2 min. In this way, the incubation is completed in the first reactor and the second reactor alternately, and the time interval between the time when discharge of the incubated biological sample from the first reactor is completed and the time when discharge of the incubated biological sample from the second reactor starts is 2 min. In other words, in this case, the reactor array, taken as a whole, outputs the incubated biological sample for 3 min every 2 min. In yet other cases, the pumping system can be so configured that the time required to completely move the biological sample out of the first reactor/second reactor after the incubation is 4 min, and the time required to completely move the biological sample to be incubated into the first reactor/second reactor is 1 min. In this way, the incubation is completed in the first reactor and the second reactor alternately, and the time interval between the time when discharge of the incubated biological sample from the first reactor is completed and the time when discharge of the incubated biological sample from the second reactor starts is 1 min. In other words, in this case, the reactor array, taken as a whole, outputs the incubated biological sample for 4 min every 1 min.

In another embodiment, the treatment timing sequence of the input, reaction and output operations performed on the two reactors can be as shown in Table 2.

**Table 2**

| Time period | First Reactor | Second reactor |
|---|---|---|
| T0 | Input + reaction | |
| T1 | Output | Input + reaction |
| T2 | Input + reaction | Output |
| T3 | Output | Input + reaction |
| T4 | Input + reaction | Output |
| T5 | Output | Input + reaction |
| T6 | Input + reaction | Output |
| ... | ... | ... |
| | | |

In an example, the time periods T0, T1, T2, ... may each be, for example, 5 min. From the timing sequence of operations in Table 2, it is known that the first reactor and the second reactor repeat "input + reaction" and "output" in adjacent timing sequences, respectively, in addition to the initial time period of T0. In some cases, the time required to complete the predetermined reaction (e.g., incubation) in the first reactor/second reactor may be, for example, 3 min. The pumping system can be so configured that the time required to completely move the biological sample to be incubated into the first reactor/second reactor is 2 min, and the time required to completely move the biological sample out of the first reactor/second reactor after the incubation is 5 min. In this way, the incubation is completed in the first reactor and the second reactor alternately, and the time interval between the time when discharge of the incubated biological sample from the first reactor is completed and the time when discharge of the incubated biological sample from the second reactor starts is zero. In other words, in this case, the reactor array, taken as a whole, outputs the incubated biological sample without interruption and continuously. For example, in each time period starting from T1, the first reactor and the second reactor discharge the reacted biological sample alternately.

In an example, as shown in FIG. 2, the system for treating a biological sample according to the present disclosure can be used for pre-denaturation treatment before nucleic acid capping.

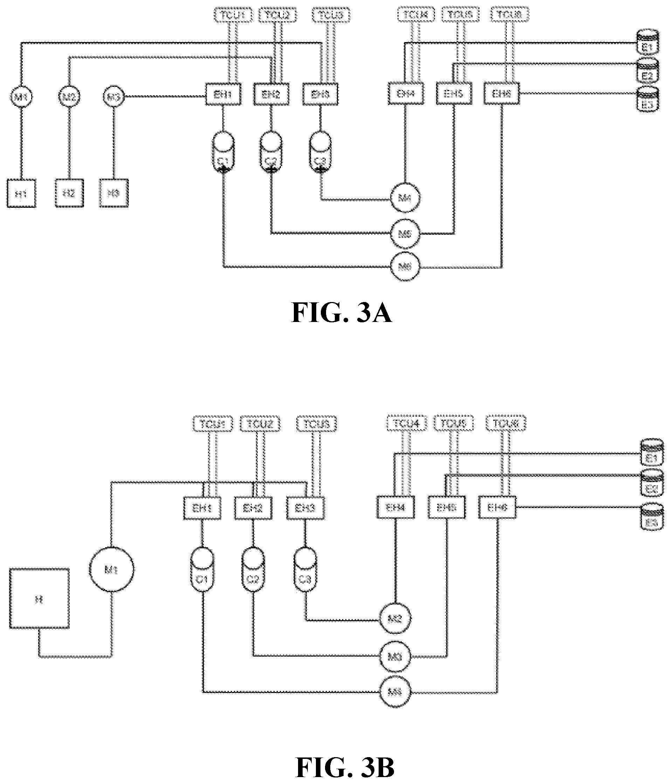

In an example, as shown in FIG. 3A or 3B , the reactor array in the system for treating a biological sample according to the present disclosure may include three reactors C1, C2 and C3. In the example shown in FIG. 3A, a separate biological sample source, a separate pumping system, and a separate biological sample collector are provided for each of the three reactors. For example, for the reactor C1, separate pumps M3 and M6 are provided. The biological sample to be treated is transferred by the pump M3 from a biological sample source H3 via a first temperature regulation device EH1 in the temperature regulation assembly to the reactor C1 (hereinafter also referred to as "input"), and undergoes a predetermined reaction (hereinafter also referred to as "reaction", e.g., incubation) in the reactor C1. After the reaction is completed, the biological sample is transferred from the reactor C1 by the pump M6 to a biological sample collector E3 (hereinafter also referred to as "output") via a second temperature regulation device EH6 in the temperature regulation assembly. The first temperature regulation device EH1 may be, for example, a temperature increasing device, and the second temperature regulation device EH6 may be, for example, a temperature decreasing device. Temperature controllers TCU1 to TCU6 may be provided for the temperature regulation devices EH1 to EH6, respectively. In the example shown in FIG. 3B, a common biological sample source, a common input pump, separate output pumps, and separate biological sample collectors are provided for the three reactors in the reactor array. For example, for the reactor C1, the biological sample to be treated is transferred from the common biological sample source H to the reactor C1 by the common input pump M1 via the first temperature regulation device EH1 in the temperature regulation assembly, and undergoes a predetermined reaction in the reactor C1. After the reaction is completed, the biological sample is transferred from the reactor C1 by the pump M4 to the biological sample collector E3 via the second temperature regulation device EH6 in the temperature regulation assembly. The first temperature regulation device EH1 may be, for example, a temperature increasing device, and the second temperature regulation device EH6 may be, for example, a temperature decreasing device. Temperature controllers TCU1 to TCU 6 may be provided for the temperature regulation devices EH1 to EH6, respectively. Although FIGS. 3A and 3B show that a separate first temperature regulation device and a separate second temperature regulation device are provided for each of the three reactors, as described above, a common first temperature regulation device and a common second temperature regulation device may be provided for the three reactors, or a common first temperature regulation device and a common second temperature regulation device may be provided for any two of the three reactors.

In an embodiment, the treatment timing sequence of the input, reaction and output operations performed on the three reactors can be as shown in Table 3.

**Table 3**

| Time period | First Reactor | Second reactor | Third reactor |
|---|---|---|---|
| T0 | Input | | |
| T1 | Reaction | Input | |
| T2 | Output | Reaction | Input |
| T3 | Input | Output | Reaction |
| T4 | Reaction | Input | Output |
| T5 | Output | Reaction | Input |
| T6 | Input | Output | Reaction |
| T7 | Reaction | Input | Output |
| T8 | Output | Reaction | Input |
| T9 | Input | Output | Reaction |
| ... | ... | ... | ... |
| | | | |

In an example, the time periods T0, T1, T2, ... may each be, for example, 5 min. From the timing sequence of operations in Table 3, it is known that the first reactor, the second reactor and the third reaction repeat "input", "reaction" and "output" in adjacent timing sequences, respectively, in addition to the two initial time periods of T0 and T1. The time required to complete the predetermined reaction (e.g., incubation) in the first reactor, the second reactor and the third reaction may be 5 min. The pumping system can be so configured that the time required to completely move the biological sample to be incubated into the first reactor, the second reactor or the third reactor is 5 min, and the time required to completely move the biological sample out of the first reactor, the second reactor, the third reactor after the incubation is 5 min. In this way, incubation is completed in the first reactor, the second reactor, and the third reactor alternately, and the time interval between the time when discharge of the incubated biological sample from the first reactor is completed and the time when discharge of the incubated biological sample from the second reactor starts is zero, the time interval between the time when discharge of the incubated biological sample from the second reactor is completed and the time when discharge of the incubated biological sample from the third reactor starts is zero, and the time interval between the time when discharge of the incubated biological sample from the third reactor is completed and the time when discharge of the incubated biological sample from the first reactor starts is zero. In other words, in this case, the reactor array, taken as a whole, outputs the incubated biological sample without interruption and continuously. For example, in each time period starting from T2, the first reactor, the second reactor and the third reactor discharge the reacted biological sample alternately.

In an embodiment, after the biological sample that has completed the previous reaction is discharged from the reactor, the inside of the reactor needs to be flushed before the next input of the biological sample. Flushing the interior of the reactor may include purging the interior of the reactor with a liquid or a gas (e.g., an inert gas). The treatment timing sequence of the input, reaction, output and flushing operations for the three reactors can be as shown in Table 4.

**Table 4**

| Time period | First Reactor | Second reactor | Third reactor |
|---|---|---|---|
| T0 | Input | | |
| T1 | Reaction | Input | |
| T2 | Output + flush | Reaction | Input |
| T3 | Input | Output + flush | Reaction |
| T4 | Reaction | Input | Output + flush |
| T5 | Output + flush | Reaction | Input |
| T6 | Input | Output + flush | Reaction |
| T7 | Reaction | Input | Output + flush |
| T8 | Output + flush | Reaction | Input |
| T9 | Input | Output + flush | Reaction |
| ... | ... | ... | ... |
| | | | |

In an example, the time periods T0, T1, T2, ... may each be, for example, 5 min. From the timing sequence of operations in Table 4, it is known that the first reactor, the second reactor and the third reaction repeat "input", "reaction" and "output + flush" in adjacent timing sequences, respectively, in addition to the two initial time periods of T0 and T1. The time required to complete the predetermined reaction (e.g., incubation) in the first reactor, the second reactor and the third reaction may be 5 min. The pumping system can be so configured that the time required to completely move the biological sample to be incubated into the first to third reactors is 5 min, and the time required to completely move the biological sample out of each of the first to third reactors after incubation is 4 min. The time required to flush each of the first to third reactors may be 1 min. In this way, incubation is completed in the first reactor, the second reactor, and the third reactor alternately, and the time interval between the time when discharge of the incubated biological sample from the first reactor is completed and the time when discharge of the incubated biological sample from the second reactor starts is 1 min, the time interval between the time when discharge of the incubated biological sample from the second reactor is completed and the time when discharge of the incubated biological sample from the third reactor starts is 1 min, and the time interval between the time when discharge of the incubated biological sample from the third reactor is completed and the time when discharge of the incubated biological sample from the first reactor starts is 1 min. In other words, in this case, the reactor array, taken as a whole, outputs the incubated biological sample for 4 min every 1 min.

In another embodiment, after the biological sample that has completed the previous reaction is discharged from the reactor, it is necessary to flush the inside of the reactor before the next input of the biological sample. The treatment timing sequence of the input, reaction, output, and flushing operations of the three reactors can also be as shown in Table 5.

**Table 5**

| Time period | First Reactor | Second reactor | Third reactor |
|---|---|---|---|
| T0 | Input | | |
| T1 | Reaction | Input | |
| T2 | Output | Reaction | Input |
| T3 | Flush + input | Output | Reaction |
| T4 | Reaction | Flush + input | Output |
| T5 | Output | Reaction | Flush + input |
| T6 | Flush + input | Output | Reaction |
| T7 | Reaction | Flush + input | Output |
| T8 | Output | Reaction | Flush + input |
| T9 | Flush + input | Output | Reaction |
| ... | ... | ... | ... |
| | | | |

In an example, the time periods T0, T1, T2, ... may each be, for example, 5 min. From the timing sequence of operations in Table 5, it is known that the first reactor, the second reactor and the third reaction repeat "flush + input", "reaction" and "output" in adjacent timing sequences, respectively, in addition to the two initial time periods of T0 and T1. The time required to complete the predetermined reaction (e.g., incubation) in the first reactor, the second reactor and the third reaction may be 5 min. The pumping system can be so configured that the time required to completely move the biological sample to be incubated into the first to third reactors is 4 min, and the time required to completely move the biological sample out of each of the first to third reactors after incubation is 5 min. The time required to flush each of the first to third reactors may be 1 min. In this way, incubation is completed in the first reactor, the second reactor, and the third reactor alternately, and the time interval between the time when discharge of the incubated biological sample from the first reactor is completed and the time when discharge of the incubated biological sample from the second reactor starts is zero, the time interval between the time when discharge of the incubated biological sample from the second reactor is completed and the time when discharge of the incubated biological sample from the third reactor starts is zero, and the time interval between the time when discharge of the incubated biological sample from the third reactor is completed and the time when discharge of the incubated biological sample from the first reactor starts is zero. In other words, in this case, the reactor array, taken as a whole, outputs the incubated biological sample continuously.

In yet another embodiment, after the biological sample that has completed the previous reaction is discharged from the reactor, it is necessary to flush the inside of the reactor before the next input of the biological sample. The treatment timing sequence of the input, reaction, output, and flushing operations of the three reactors can also be as shown in Table 6.

**Table 6**

| Time period | First Reactor | Second reactor | Third reactor |
|---|---|---|---|
| T0 | Input | | |
| T1 | Reaction | Input | |
| T2 | Output | Reaction | Input |
| T3 | Flush | Output | Reaction |
| T4 | Input | Flush | Output |
| T5 | Reaction | Input | Flush |
| T6 | Output | Reaction | Input |
| T7 | Flush | Output | Reaction |
| T8 | Input | Flush | Output |
| T9 | Reaction | Input | Flush |
| ... | ... | ... | ... |
| | | | |

In an example, the time periods T0, T1, T2, ... may each be, for example, 5 min. From the timing sequence of operations in Table 6, it is known that the first reactor, the second reactor and the third reaction repeat "input", "reaction", "output" and "flush" in adjacent timing sequences, respectively, in addition to the two initial time periods of T0 and T1. The time required to complete the predetermined reaction (e.g., incubation) in the first reactor, the second reactor and the third reaction may be 5 min. The pumping system can be so configured that the time required to completely move the biological sample to be incubated into the first to third reactors is 5 min, and the time required to completely move the biological sample out of each of the first to third reactors after incubation is 5 min. The time required to flush each of the first to third reactors may be 5 min or less than 5 min. In this way, incubation is completed in the first reactor, the second reactor, and the third reactor alternately, and the time interval between the time when discharge of the incubated biological sample from the first reactor is completed and the time when discharge of the incubated biological sample from the second reactor starts is zero, the time interval between the time when discharge of the incubated biological sample from the second reactor is completed and the time when discharge of the incubated biological sample from the third reactor starts is zero, and the time interval between the time when discharge of the incubated biological sample from the third reactor is completed and the time when discharge of the incubated biological sample from the first reactor starts is 5 min. In other words, in this case, the reactor array, taken as a whole, outputs the incubated biological sample continuously for 15 min every 5 min.

As described with reference to the examples in Tables 5 and 6, where additional treatment is required (e.g., flushing described above, or detection and confirmation of reaction environment parameters in a reactor), the additional treatment may be carried out in combination with or separately from any of the three basic treatments "input", "reaction" and "output", while achieving "the time interval between the time when discharge of the biological sample having undergone a predetermined treatment from a first reactor is completed and the time when discharge of the biological sample having undergone a predetermined treatment from a second reactor immediately next to the first reactor starts is less than a predetermined time period" as described in the present disclosure.

In an example, the reactor array in the system for treating a biological sample according to the present disclosure may include four reactors. A separate biological sample source, a separate pumping system, and a separate biological sample collector may be provided for each of the four reactors. It is also possible to provide a common biological sample source, a common pumping system and a common biological sample collector for the four reactors. In an embodiment, the treatment timing sequence of the input, reaction and output operations performed on the four reactors can be as shown in Table 7.

**Table 7**

| Time period | First Reactor | Second reactor | Third reactor | Fourth reactor |
|---|---|---|---|---|
| T0 | Input | | | |
| T1 | 1/2 reaction time | Input | | |
| T2 | Total reaction time | 1/2 reaction time | Input | |
| T3 | Output | Total reaction time | 1/2 reaction time | Input |
| T4 | Input | Output | Total reaction time | 1/2 reaction time |
| T5 | 1/2 reaction time | Input | Output | Total reaction time |
| T6 | Total reaction time | 1/2 reaction time | Input | Output |
| T7 | Output | Total reaction time | 1/2 reaction time | Input |
| T8 | Input | Output | Total reaction time | 1/2 reaction time |
| T9 | 1/2 reaction time | Input | Output | Total reaction time |
| ... | ... | ... | ... | ... |
| | | | | |

In an example, the time periods T0, T1, T2, ... may each be, for example, 5 min. From the timing sequence of operations in Table 7, it is known that the first to fourth reactors repeat "input", "1/2 reaction time", "total reaction time" and "output" in adjacent timing sequences, respectively, in addition to the three initial time periods of T0-T2. The time required to complete the predetermined reaction (e.g., incubation) in the first to fourth reactors may be 10 min. The pumping system can be so configured that the time required to completely move the biological sample to be incubated into the first to fourth reactors is 5 min, and the time required to completely move the biological sample out of each of the first to fourth reactors after incubation is 5 min. In this way, incubation is completed in the first to fourth reactors alternately, and the time interval between the time when discharge of the incubated biological sample from the first reactor is completed and the time when discharge of the incubated biological sample from the second reactor starts is zero, the time interval between the time when discharge of the incubated biological sample from the second reactor is completed and the time when discharge of the incubated biological sample from the third reactor starts is zero, the time interval between the time when discharge of the incubated biological sample from the third reactor is completed and the time when discharge of the incubated biological sample from the fourth reactor starts is zero, and the time interval between the time when discharge of the incubated biological sample from the fourth reactor is completed and the time when discharge of the incubated biological sample from the first reactor starts is zero. In other words, in this case, the reactor array, taken as a whole, outputs the incubated biological sample without interruption and continuously. For example, in each time period starting from T3, the first reactor, the second reactor, the third reactor and the fourth reactor discharge the reacted biological sample alternately.

In an example, the reactor array in the system for treating a biological sample according to the present disclosure may include five reactors. A separate biological sample source, a separate pumping system, and a separate biological sample collector may be provided for each of the five reactors. It is also possible to provide a common biological sample source, a common pumping system and a common biological sample collector for the five reactors. In an embodiment, the treatment timing sequence of the input, reaction and output operations performed on the five reactors can be as shown in Table 8.

**Table 8**

| Time period | First Reactor | Second reactor | Third reactor | Fourth reactor | Fifth reactor |
|---|---|---|---|---|---|
| T0 | Input | | | | |
| T1 | 1/3 reaction time | Input | | | |
| T2 | 2/3 reaction time | 1/3 reaction time | Input | | |
| T3 | Total | 2/3 | 1/3 | Input | |
| | reaction time | reaction time | reaction time | | |
| T4 | Output | Total reaction time | 2/3 reaction time | 1/3 reaction time | Input |
| T5 | Input | Output | Total reaction time | 2/3 reaction time | 1/3 reaction time |
| T6 | 1/3 reaction time | Input | Output | Total reaction time | 2/3 reaction time |
| T7 | 2/3 reaction time | 1/3 reaction time | Input | Output | Total reaction time |
| T8 | Total reaction time | 2/3 reaction time | 1/3 reaction time | Input | Output |
| T9 | Output | Total reaction time | 2/3 reaction time | 1/3 reaction time | Input |
| ... | ... | ... | ... | ... | ... |
| | | | | | |

In an example, the time periods T0, T1, T2, ... may each be, for example, 2 min. From the timing sequence of operations in Table 8, it is known that the first to fifth reactors repeat "input", "1/3 reaction time", "2/3 reaction time", "total reaction time" and "output" in adjacent timing sequences, respectively, in addition to the three initial time periods of T0-T3. The time required to complete the predetermined reaction (e.g., incubation) in the first to fifth reactors may be 6 min. The pumping system can be so configured that the time required to completely move the biological sample to be incubated into the first to fifth reactors is 2 min, and the time required to completely move the biological sample out of each of the first to fifth reactors after incubation is 2 min. In this way, incubation is completed in the first to fifth reactors alternately, and the time interval between the time when discharge of the incubated biological sample from the first reactor is completed and the time when discharge of the incubated biological sample from the second reactor starts is zero, the time interval between the time when discharge of the incubated biological sample from the second reactor is completed and the time when discharge of the incubated biological sample from the third reactor starts is zero, the time interval between the time when discharge of the incubated biological sample from the third reactor is completed and the time when discharge of the incubated biological sample from the fourth reactor starts is zero, the time interval between the time when discharge of the incubated biological sample from the fourth reactor is completed and the time when discharge of the incubated biological sample from the fifth reactor starts is zero, and the time interval between the time when discharge of the incubated biological sample from the fifth reactor is completed and the time when discharge of the incubated biological sample from the first reactor starts is zero. In other words, in this case, the reactor array, taken as a whole, outputs the incubated biological sample without interruption and continuously. For example, in each time period starting from T4, the first reactor, the second reactor, the third reactor, the fourth reactor and the fifth reactor discharge the reacted biological sample alternately.

From the illustrative examples described above with reference to Tables 1 to 8, it can be seen that by coordinating the time for performing a predetermined reaction in each reactor in the reactor array (e.g., the start time and the end time of the reaction), the time interval between the time for completing the predetermined reaction in two immediately adjacent reactors can be made less than a predetermined time period. For example, by carrying out predetermined reactions in the reactors alternately and discharging the biological samples after the reactions alternately, it is possible to achieve what is stated in the present disclosure: "the time interval between the time when discharge of the biological sample having undergone a predetermined treatment from a first reactor is completed and the time when discharge of the biological sample having undergone a predetermined treatment from a second reactor immediately next to the first reactor starts is less than a predetermined time period." This can be achieved by coordinating and controlling at least one of the following parameters: the capacity of the reactors in the reactor array, the time required to complete a predetermined reaction in the reactor, the time required to completely transfer the biological sample to the reactors, the time required to completely transfer the biological sample from the reactors to other components, the time required to perform other treatments in the reactor, etc.

Compared to existing systems having a large-capacity reactor (e.g., 10 L), the system for treating a biological sample according to the present disclosure has two or more small-capacity reactors (e.g., 1.5 L). The timing sequence of performing (e.g., starting and completing) the predetermined reactions in the reactors of the system of the present disclosure is coordinated and controlled, so that the predetermined reactions are alternately performed and completed in the reactors and the biological samples after the reactions are alternately discharged. Taking the incubation reaction in the system as an example, since the time required for heating and cooling a smaller amount of biological samples is much shorter than the time required for the same operation on a larger amount of biological samples, the system of the present disclosure shortens the time of each heating-reaction-cooling cycle, thereby reducing the waiting time before each discharge of the reacted biological sample. In some cases, the reactor array, taken as a whole, can output the incubated biological sample without interruption and continuously.

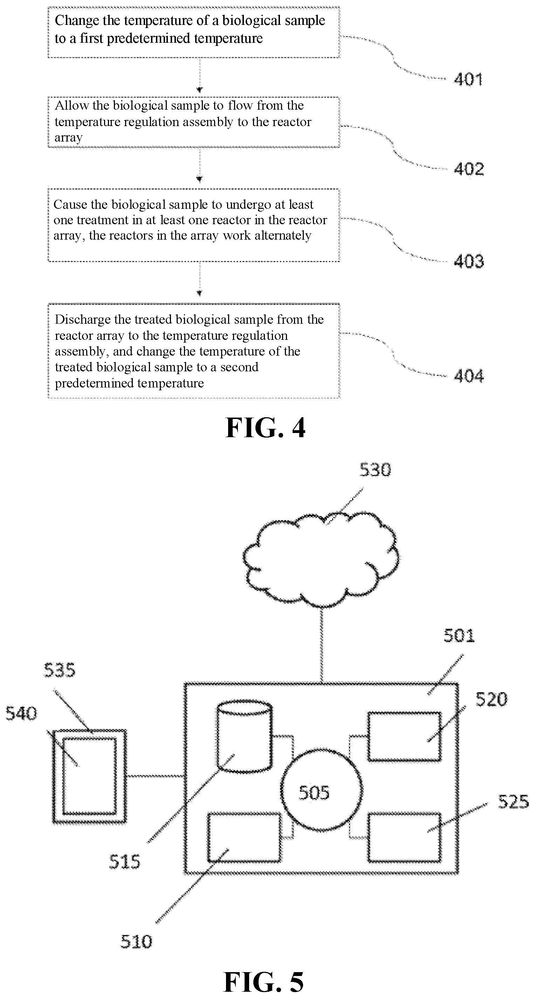

FIG. 4 is a flow chart depicting an exemplary method for treating a biological sample according to the present disclosure. In treatment 401, the temperature of a biological sample is changed to a first predetermined temperature by a temperature regulation assembly. This can be achieved by making the biological sample to be treated flow from the biological sample source through the heating device of the temperature regulation assembly by using a pumping system. For example, under the control of the temperature control device, the heating device of the temperature regulation assembly heats the biological sample to be treated to a first predetermined temperature (e.g., 65°C) within a predetermined time period (e.g., less than 10 s). In treatment 402, the heated biological sample is allowed to flow from the temperature regulation assembly to the reactor array. The reactor array may include two or more reactors, which may be arranged in parallel. Each reactor has an input port configured to receive a biological sample and an output port configured to discharge the treated biological sample. In some cases, the input port and the output port may be the same port, which is used as an input port in a first time period and as an output port in a second time period through time division multiplex access.

In treatment 403, the biological sample undergoes at least one treatment in at least one reactor in the reactor array. In an example, the at least one treatment comprises incubation. In some cases, the reaction conditions of the reactor (e.g., the capacity, temperature, pressure of the reactor) can be adjusted so that the at least one treatment of the biological sample is completed within a time period that is less than a predetermined time period. The predetermined time period may not exceed 5 min. In treatment 404, the treated biological sample is discharged from the reactor array to the temperature regulation assembly, and the temperature of the treated biological sample is changed to a second predetermined temperature using the temperature regulation assembly. This can be achieved by using a pumping system to make the treated biological sample flow from the reactor through the cooling device of the temperature regulation assembly. For example, under the control of the temperature control device, the cooling device of the temperature regulation assembly lowers the temperature of the treated biological sample to a second predetermined temperature (e.g., 2-8°C) within a predetermined time period (e.g., less than 10 s). As described above, by carrying out reactions in the reactors in the reactor array alternately and discharging the biological samples after the reactions alternately, it is possible to achieve the requirement that the time interval between the time when discharge of the biological sample having undergone a predetermined treatment from a first reactor is completed and the time when discharge of the biological sample having undergone a predetermined treatment from a second reactor immediately next to the first reactor starts is less than a predetermined time period. In some cases, the reactor array, taken as a whole, can output the incubated biological sample without interruption and continuously as described above in the present disclosure.

The exemplary method for treating a biological sample of the present disclosure shown in FIG. 4 can be executed by a computer system. The present disclosure also provides a computer system programmed to perform the method of the present disclosure. FIG. 5 shows a computer system 501 programmed or otherwise configured to implement the sample collection method or sample treatment method provided by the present disclosure. The computer system may facilitate various aspects of the methods of the present disclosure. The computer system 501 may be a user's electronic device or a computer system located remotely from the electronic device. The electronic device may be a mobile electronic device.

The computer system 501 includes a central processing unit (CPU, also referred to herein as a "processor" and a "computer processor") 505, which may be a single-core or multi-core processor, or a plurality of processors for parallel processing. The computer system 501 also includes a memory or memory location 510 (e.g., random access memory, read-only memory, flash memory), an electronic storage unit 515 (e.g., hard disk), communication interface 520 (e.g., network adapters) for communicating with one or more other systems, and peripheral devices 525, such as cache memory, other memories, data storage, and/or electronic display adapters. The memory 510, the storage unit 515, the interface 520, and the peripheral device 525 communicate with the CPU 505 through a communication bus (solid lines) such as the mainboard. The storage unit 515 may be a data storage unit (or a data repository) for storing data. The computer system 501 may be operably coupled to a computer network ("network") 530 by means of the communication interface 520. The network 530 may be the Internet, the internetwork and/or an extranet, or an intranet and/or an extranet in communication with the Internet. In some cases, the network 530 is a telecommunications and/or data network. The network 530 may include one or more computer servers, which may enable distributed computing, such as cloud computing. In some cases, the network 530 may implement a peer-to-peer network with the help of the computer system 501, which may enable devices coupled to the computer system 501 to act as clients or servers.

The CPU 505 can execute a series of machine-readable instructions, which can be embodied in a program or software. The instructions may be stored in a memory location such as memory 510. The instructions may be directed to the CPU 505, which may then program or otherwise configure the CPU 505 to implement the method of the present disclosure. Examples of operations performed by the CPU 505 may include fetching, decoding, executing, and writing back.

The CPU 505 may be part of a circuit such as an integrated circuit. One or more other components of the system 501 may be included in the circuit. In some cases, the circuit is an application-specific integrated circuit (ASIC).

The storage unit 515 can store files such as drivers, libraries, and saved programs. The storage unit 515 may store user data, such as user preferences and user programs. In some cases, the computer system 501 may include one or more additional data storage units located external to the computer system 501, such as on a remote server that communicates with the computer system 501 via an intranet or the Internet.

The computer system 501 can communicate with one or more remote computer systems via the network 530. For example, the computer system 501 may communicate with a user's remote computer system. Examples of remote computer systems include a personal computer (e.g., a laptop PC), a tablet PC or tablet computer (e.g., Apple^{®} iPad, Samsung^{®} Galaxy Tab), a phone, a smartphone (e.g., Apple^{®} iPhone, an Android-enabled device, a Blackberry^{®}), or a personal digital assistant. Users can access the computer system 501 through the network 530.

The methods described herein may be implemented by machine (e.g., computer processor) executable codes stored in an electronic storage location (e.g., memory 510 or electronic storage unit 515) of the computer system 501. Machine executable or machine readable codes may be provided in the form of software. During use, the code may be executed by the processor 505. In some cases, codes may be retrieved from the storage unit 515 and stored in the memory 510 for ready access by the processor 505. In some cases, the electronic storage unit 515 may be eliminated and machine-executable instructions may be stored in the memory 510.

The code may be pre-compiled and configured for use with a machine having a processor suitable for executing the code, or may be compiled during runtime. The code may be provided in a programming language which may be selected so that the code can be executed in a pre-compiled or as-compiled manner.

Various aspects of the systems and methods provided herein (e.g., the computer system 501) may be embodied in programming. Aspects of the technology may be considered as "products" or "artifacts" in the form of machine (or processor) executable codes and/or associated data that are typically carried on machine-readable media or embodied in the type of machine-readable media. The machine executable code may be stored in an electronic storage unit, such as a memory (e.g., read-only memory, random access memory, flash memory) or a hard disk. The "storage" type of media may include any or all tangible memories of a computer, processor, etc., or related modules thereof, such as various semiconductor memories, tape drives, disk drives, which can provide non-transitory storage for software programming at any time. All or portions of the software may from time to time be communicated over the Internet or other various telecommunications networks. For example, such communications may enable software to be loaded from one computer or processor to another, such as from a management server or host to an application server's computer platform. Thus, another type of media that can carry software elements includes optical, radio, and electromagnetic waves, such as those used over physical interfaces between local devices through wired and optical landline networks and through various air-links. The physical elements that carry such waves, such as wired or wireless links, optical links, can also be considered as the medium that carries the software. As used herein, unless limited to non-transitory tangible "storage" media, the term "readable media", such as a computer or machine, refers to any media involved in providing instructions to a processor for execution.

Therefore, a machine-readable medium such as a computer executable code may take a variety of forms, including but not limited to a tangible storage medium, a carrier medium, or a physical transmission medium. The non-volatile storage medium includes, for example, an optical disk or a magnetic disk, for example, any storage device in any computer, which can be used to implement the database shown in the drawings, for example. Volatile storage media include dynamic memories, such as the main memory of such a computer platform. Tangible transmission media include coaxial cables; copper wire and fiber optics, including the wires that make up the bus within a computer system. Carrier transmission media can take the form of electric or electromagnetic signals, or acoustic or optical waves, such as those generated during radio frequency (RF) and infrared (IR) data communications. Thus, common forms of computer readable media include, for example, floppy disk, floppy magnetic disk, hard disk, magnetic tape, any other magnetic medium, CD-ROM, DVD or DVD-ROM, any other optical medium, punch cardboard magnetic tape, any other physical storage medium with a pattern of holes, RAM, ROM, PROM and EPROM, FLASH-EPROM, any other memory chip or cartridge, carrier that transports data or instructions, a cable or link that transports such a carrier, or any other medium from which a computer can read programming codes and/or data. Many of these forms of computer readable media may be involved in carrying one or more sequences of one or more instructions to a processor for execution.

The computer system 501 may include or be in communication with an electronic display 535, the electronic display 535 includes a user interface (UI) 540, and the user interface (UI) 540 is used for providing, for example, test conditions and protocols. Examples of UI include, but are not limited to, graphical user interfaces (GUIs) and web-based user interfaces.

The present disclosure provides the following exemplary but non-limiting embodiments.
1. A system for treating a biological sample, the system comprising:
   a temperature regulation assembly configured to change the temperature of a biological sample flowing therethrough; and
   a reactor array coupled to the temperature regulation assembly, the reactor array comprising two or more reactors, each reactor in the reactor array being configured to cause the biological sample to undergo at least one treatment, each of the reactors having an input port configured to receive the biological sample and an output port configured to discharge the treated biological sample,
   wherein the treatment is performed in the two or more reactors alternately, such that the time interval between completion of discharge of the treated biological sample from a first reactor and start of discharge of the treated biological sample from a second reactor is less than a predetermined time period, and the second reactor is immediately next to the first reactor in the reactor array.
2. The system according to embodiment 1, wherein the predetermined time period is less than five minutes.
3. The system according to embodiment 2, wherein the predetermined time period is less than one minute.
4. The system according to embodiment 3, wherein the predetermined time period is less than 30 s.
5. The system according to embodiment 1, wherein the two or more reactors are connected in parallel with each other.
6. The system according to embodiment 1, wherein the volume of each reactor in the reactor array does not exceed 5 L.
7. The system according to embodiment 6, wherein the volume of each reactor in the reactor array does not exceed 3 L.
8. The system according to embodiment 7, wherein the volume of each reactor in the reactor array does not exceed 1.5 L.
9. The system according to embodiment 1, wherein the temperature regulation assembly comprises a first temperature regulation device, and the first temperature regulation device is configured to increase the temperature of the biological sample to a first predetermined temperature within a first time period.
10. The system according to embodiment 9, wherein the first time period does not exceed 5 min.
11. The system according to embodiment 10, wherein the first time period does not exceed 1 min.
12. The system according to embodiment 9, wherein the temperature regulation assembly comprises a second temperature regulation device, and the second temperature regulation device is configured to decrease the temperature of the biological sample to a second predetermined temperature within a second time period.
13. The system according to embodiment 12, wherein the second time period does not exceed 5 min.
14. The system according to embodiment 13, wherein the second time period does not exceed 1 min.
15. The system according to embodiment 1, wherein each of the reactors is configured to treat the biological sample at a predetermined treatment temperature.
16. The system according to embodiment 15, wherein a temperature adjustment device is provided for each of the reactors.
17. The system according to embodiment 1, wherein at least one reactor in the reactor array comprises an incubator.
18. The system according to embodiment 1, wherein the biological sample comprises nucleic acid.
19. The system according to embodiment 18, wherein the at least one treatment comprises nucleic acid denaturation.
20. The system according to embodiment 1, further comprising a biological sample source coupled to the temperature regulation assembly.
21. The system according to embodiment 1, further comprising a pumping system configured to perform at least one of the following operations: (i) transfer the biological sample from a biological sample source to the temperature regulation assembly, (ii) transfer the biological sample from the temperature regulation assembly to at least one reactor in the reactor array, and (iii) transfer the biological sample from at least one reactor in the reactor array to the temperature regulation assembly.
22. The system according to embodiment 1, further comprising a sensing system coupled to the reactor array, wherein the sensing system is configured to sense at least one parameter of at least one reactor in the reactor array.
23. The system according to embodiment 22, wherein the at least one parameter includes at least one of the temperature, pressure, and weight of the biological sample in the at least one reactor.
24. The system according to embodiment 1, further comprising a waste liquid container coupled to the reactor array, wherein the waste liquid container is configured to store waste liquid from at least one reactor in the reactor array.
25. The system according to embodiment 1, further comprising a flushing device configured to flush the interior of at least one reactor in the reactor array.
26. The system according to embodiment 25, wherein the flushing device uses a gas or a liquid to flush the interior of at least one reactor in the reactor array.
27. A method for treating a biological sample, the method comprising:
   changing the temperature of a biological sample to a first predetermined temperature using a temperature regulation assembly;
   allowing the biological sample to flow from the temperature regulation assembly to a reactor array, the reactor array comprising two or more reactors, each of the reactors having an input port configured to receive the biological sample and an output port configured to discharge the treated biological sample;
   causing the biological sample to undergo at least one treatment in at least one reactor in the reactor array; and
   discharging the treated biological sample from the reactor array to the temperature regulation assembly, and changing the temperature of the treated biological sample to a second predetermined temperature using the temperature regulation assembly,
   wherein the treatment is performed in the two or more reactors alternately, such that the time interval between completion of discharge of the treated biological sample from a first reactor and start of discharge of the treated biological sample from a second reactor is less than a predetermined time period, and the second reactor is immediately next to the first reactor in the reactor array.
28. The method according to embodiment 27, wherein the predetermined time period is less than five minutes.
29. The method according to embodiment 28, wherein the predetermined time period is less than one minute.
30. The method according to embodiment 29, wherein the predetermined time period is less than 30 seconds.
31. The method according to embodiment 27, wherein the two or more reactors are connected in parallel with each other.
32. The method according to embodiment 27, wherein the volume of each reactor in the reactor array does not exceed 5 L.
33. The method according to embodiment 32, wherein the volume of each reactor in the reactor array does not exceed 3 L.
34. The method according to embodiment 33, wherein the volume of each reactor in the reactor array does not exceed 1.5 L.
35. The method according to embodiment 27, wherein changing the temperature of a biological sample to a first predetermined temperature using a temperature regulation assembly comprises raising the temperature of the biological sample to the first predetermined temperature using a first temperature regulation device of the temperature regulation assembly within a first time period.
36. The method according to embodiment 35, wherein the first time period does not exceed 5 min.
37. The method according to embodiment 36, wherein the first time period does not exceed 1 min.
38. The method according to embodiment 35, wherein changing the temperature of the treated biological sample to a second predetermined temperature using the temperature regulation assembly comprises lowering the temperature of the biological sample to the second predetermined temperature using a second temperature regulation device of the temperature regulation assembly within a second time period.
39. The method according to embodiment 38, wherein the second time period does not exceed 5 min.
40. The method according to embodiment 39, wherein the second time period does not exceed 1 min.
41. The method according to embodiment 27, wherein causing the biological sample to undergo at least one treatment in at least one reactor in the reactor array comprises treating the biological sample at a predetermined treatment temperature.
42. The method according to embodiment 41, wherein each of the reactors is provided with a temperature adjustment device.
43. The method according to embodiment 27, wherein at least one reactor in the reactor array comprises an incubator.
44. The method according to embodiment 27, wherein the biological sample comprises nucleic acid.
45. The method according to embodiment 44, wherein the at least one treatment comprises nucleic acid denaturation.
46. The method according to embodiment 27, further comprising transferring the biological sample from a biological sample source to the temperature regulation assembly.
47. The method according to embodiment 27, further comprising performing at least one of the following operations using a pumping system: (i) transfer the biological sample from a biological sample source to the temperature regulation assembly, (ii) transfer the biological sample from the temperature regulation assembly to at least one reactor in the reactor array, and (iii) transfer the biological sample from at least one reactor in the reactor array to the temperature regulation assembly.
48. The method according to embodiment 27, further comprising sensing at least one parameter of at least one reactor in the reactor array using a sensing system coupled to the reactor array.
49. The method according to embodiment 48, wherein the at least one parameter includes at least one of the temperature, pressure, and weight of the biological sample in the at least one reactor.
50. The method according to embodiment 27, further comprising storing waste liquid from at least one reactor in the reactor array in a waste liquid container coupled to the reactor array.
51. The method according to embodiment 27, further comprising flushing the interior of at least one reactor in the reactor array using a flushing device.
52. The method according to embodiment 51, wherein the flushing device uses a gas or a liquid to flush the interior of at least one reactor in the reactor array.
53. A system for treating a biological sample, the system comprising one or more processors and a non-volatile memory storing computer executable instructions, wherein when executing the computer executable instructions, the one or more processors perform a method, the method comprising:
   changing the temperature of a biological sample to a first predetermined temperature using a temperature regulation assembly;
   allowing the biological sample to flow from the temperature regulation assembly to a reactor array, the reactor array comprising two or more reactors, each of the reactors having an input port configured to receive the biological sample and an output port configured to discharge the treated biological sample;
   causing the biological sample to undergo at least one treatment in at least one reactor in the reactor array; and
   discharging the treated biological sample from the reactor array to the temperature regulation assembly, and changing the temperature of the treated biological sample to a second predetermined temperature using the temperature regulation assembly,
   wherein the treatment is performed in the two or more reactors alternately, such that the time interval between completion of discharge of the treated biological sample from a first reactor and start of discharge of the treated biological sample from a second reactor is less than a predetermined time period, and the second reactor is immediately next to the first reactor in the reactor array.
54. A non-volatile memory storing computer executable instructions, the computer executable instructions comprising:
   a module instructing to change the temperature of a biological sample to a first predetermined temperature using a temperature regulation assembly;
   a module instructing to allow the biological sample to flow from the temperature regulation assembly to a reactor array, the reactor array comprising two or more reactors, each of the reactors having an input port configured to receive the biological sample and an output port configured to discharge the treated biological sample;
   a module instructing to cause the biological sample to undergo at least one treatment in at least one reactor in the reactor array; and
   a module instructing to discharge the treated biological sample from the reactor array to the temperature regulation assembly, and change the temperature of the treated biological sample to a second predetermined temperature using the temperature regulation assembly,
   wherein the treatment is performed in the two or more reactors alternately, such that the time interval between completion of discharge of the treated biological sample from a first reactor and start of discharge of the treated biological sample from a second reactor is less than a predetermined time period, and the second reactor is immediately next to the first reactor in the reactor array.
55. A system for treating a biological sample, the system comprising:
   a temperature regulation assembly configured to change the temperature of a biological sample flowing therethrough; and
   a reactor array coupled to the temperature regulation assembly, the reactor array comprising two or more reactors, each reactor in the reactor array being configured to cause the biological sample to undergo at least one treatment, each of the reactors having an input port configured to receive the biological sample and an output port configured to discharge the treated biological sample,
   wherein the treatment in the two or more reactors is coordinated so that the time interval between completion of the treatment in a first reactor and completion of the treatment in a second reactor is less than a predetermined time period, and the second reactor is immediately next to the first reactor in the reactor array.
56. A method for treating a biological sample, the method comprising:
   changing the temperature of a biological sample to a first predetermined temperature using a temperature regulation assembly;
   allowing the biological sample to flow from the temperature regulation assembly to a reactor array, the reactor array comprising two or more reactors, each of the reactors having an input port configured to receive the biological sample and an output port configured to discharge the treated biological sample;
   causing the biological sample to undergo at least one treatment in at least one reactor in the reactor array; and
   discharging the treated biological sample from the reactor array to the temperature regulation assembly, and changing the temperature of the treated biological sample to a second predetermined temperature using the temperature regulation assembly,
   wherein the treatment in the two or more reactors is coordinated so that the time interval between completion of the treatment in a first reactor and completion of the treatment in a second reactor is less than a predetermined time period, and the second reactor is immediately next to the first reactor in the reactor array.
57. A system for treating a biological sample, the system comprising:
   a temperature regulation assembly configured to change the temperature of a biological sample flowing therethrough; and
   a reactor array coupled to the temperature regulation assembly, the reactor array comprising two or more reactors, each of the reactors having an input port configured to receive the biological sample and an output port configured to discharge the treated biological sample, wherein the treatment is performed in the two or more reactors alternately, and each reactor in the reactor array is configured to cause the biological sample to undergo at least one treatment within a time period not exceeding a predetermined time period.
58. The system according to embodiment 57, wherein the predetermined time period is less than ten minutes.
59. The system according to embodiment 58, wherein the predetermined time period is less than five minutes.
60. The system according to embodiment 57, wherein the two or more reactors are connected in parallel with each other.
61. The system according to embodiment 57, wherein the volume of each reactor in the reactor array does not exceed 5 L.
62. The system according to embodiment 61, wherein the volume of each reactor in the reactor array does not exceed 3 L.
63. The system according to embodiment 62, wherein the volume of each reactor in the reactor array does not exceed 1.5 L.
64. The system according to embodiment 57, wherein the temperature regulation assembly comprises a first temperature regulation device, and the first temperature regulation device is configured to increase the temperature of the biological sample to a first predetermined temperature within a first time period.
65. The system according to embodiment 64, wherein the first time period does not exceed 5 min.
66. The system according to embodiment 65, wherein the first time period does not exceed 1 min.
67. The system according to embodiment 64, wherein the temperature regulation assembly comprises a second temperature regulation device, and the second temperature regulation device is configured to decrease the temperature of the biological sample to a second predetermined temperature within a second time period.
68. The system according to embodiment 67, wherein the second time period does not exceed 5 min.
69. The system according to embodiment 68, wherein the second time period does not exceed 1 min.
70. The system according to embodiment 57, wherein each of the reactors is configured to treat the biological sample at a predetermined treatment temperature.
71. The system according to embodiment 70, wherein each of the reactors is provided with a temperature adjustment device.
72. The system according to embodiment 57, wherein at least one reactor in the reactor array comprises an incubator.
73. The system according to embodiment 57, wherein the biological sample comprises nucleic acid.
74. The system according to embodiment 73, wherein the at least one treatment comprises nucleic acid denaturation.
75. The system according to embodiment 57, further comprising a biological sample source coupled to the temperature regulation assembly.
76. The system according to embodiment 57, further comprising a pumping system configured to perform at least one of the following operations: (i) transfer the biological sample from a biological sample source to the temperature regulation assembly, (ii) transfer the biological sample from the temperature regulation assembly to at least one reactor in the reactor array, and (iii) transfer the biological sample from at least one reactor in the reactor array to the temperature regulation assembly.
77. The system according to embodiment 57, further comprising a sensing system coupled to the reactor array, wherein the sensing system is configured to sense at least one parameter of at least one reactor in the reactor array.
78. The system according to embodiment 77, wherein the at least one parameter includes at least one of the temperature, pressure, and weight of the biological sample in the at least one reactor.
79. The system according to embodiment 57, further comprising a waste liquid container coupled to the reactor array, wherein the waste liquid container is configured to store waste liquid from at least one reactor in the reactor array.
80. The system according to embodiment 57, further comprising a flushing device configured to flush the interior of at least one reactor in the reactor array.
81. The system according to embodiment 80, wherein the flushing device uses a gas or a liquid to flush the interior of at least one reactor in the reactor array.
82. A method for treating a biological sample, the method comprising:
   changing the temperature of a biological sample to a first predetermined temperature using a temperature regulation assembly;
   allowing the biological sample to flow from the temperature regulation assembly to a reactor array, the reactor array comprising two or more reactors, each of the reactors having an input port configured to receive the biological sample and an output port configured to discharge the treated biological sample;
   performing at least one treatment on the biological sample in the two or more reactors alternately, wherein the biological sample undergoes the at least one treatment in the at least one reactor within a time period not exceeding a predetermined time period; and
   discharging the treated biological sample from the reactor array to the temperature regulation assembly, and changing the temperature of the treated biological sample to a second predetermined temperature using the temperature regulation assembly.
83. The method according to embodiment 82, wherein the predetermined time period is less than ten minutes.
84. The method according to embodiment 83, wherein the predetermined time period is less than five minutes.
85. The method according to embodiment 82, wherein the two or more reactors are connected in parallel with each other.
86. The method according to embodiment 82, wherein the volume of each reactor in the reactor array does not exceed 5 L.
87. The method according to embodiment 86, wherein the volume of each reactor in the reactor array does not exceed 3 L.
88. The method according to embodiment 87, wherein the volume of each reactor in the reactor array does not exceed 1.5 L.
89. The method according to embodiment 82, wherein changing the temperature of a biological sample to a first predetermined temperature using a temperature regulation assembly comprises raising the temperature of the biological sample to the first predetermined temperature using a first temperature regulation device of the temperature regulation assembly within a first time period.
90. The method according to embodiment 89, wherein the first time period does not exceed 5 min.
91. The method according to embodiment 90, wherein the first time period does not exceed 1 min.
92. The method according to embodiment 89, wherein changing the temperature of the treated biological sample to a second predetermined temperature using the temperature regulation assembly comprises lowering the temperature of the biological sample to the second predetermined temperature using a second temperature regulation device of the temperature regulation assembly within a second time period.
93. The method according to embodiment 92, wherein the second time period does not exceed 5 min.
94. The method according to embodiment 93, wherein the second time period does not exceed 1 min.
95. The method according to embodiment 82, wherein causing the biological sample to undergo at least one treatment in at least one reactor in the reactor array comprises treating the biological sample at a predetermined treatment temperature.
96. The method according to embodiment 95, wherein each of the reactors is provided with a temperature adjustment device.
97. The method according to embodiment 82, wherein at least one reactor in the reactor array comprises an incubator.
98. The method according to embodiment 82, wherein the biological sample comprises nucleic acid.
99. The method according to embodiment 98, wherein the at least one treatment comprises nucleic acid denaturation.
100. The method according to embodiment 82, further comprising transferring the biological sample from a biological sample source to a temperature regulation assembly.
101. The method according to embodiment 100, further comprising performing at least one of the following operations using a pumping system: (i) transfer the biological sample from a biological sample source to the temperature regulation assembly, (ii) transfer the biological sample from the temperature regulation assembly to at least one reactor in the reactor array, and (iii) transfer the biological sample from at least one reactor in the reactor array to the temperature regulation assembly.
102. The method according to embodiment 82, further comprising sensing at least one parameter of at least one reactor in the reactor array using a sensing system coupled to the reactor array.
103. The method according to embodiment 102, wherein the at least one parameter includes at least one of the temperature, pressure, and weight of the biological sample in the at least one reactor.
104. The method according to embodiment 82, further comprising storing waste liquid from at least one reactor in the reactor array in a waste liquid container coupled to the reactor array.
105. The method according to embodiment 82, further comprising flushing the interior of at least one reactor in the reactor array using a flushing device.
106. The method according to embodiment 105, wherein the flushing device uses a gas or a liquid to flush the interior of at least one reactor in the reactor array.
107. A system for treating a biological sample, the system comprising one or more processors and a non-volatile memory storing computer executable instructions, wherein when executing the computer executable instructions, the one or more processors perform a method, the method comprising:
   changing the temperature of a biological sample to a first predetermined temperature using a temperature regulation assembly;
   allowing the biological sample to flow from the temperature regulation assembly to a reactor array, the reactor array comprising two or more reactors, each of the reactors having an input port configured to receive the biological sample and an output port configured to discharge the treated biological sample;
   performing at least one treatment on the biological sample in the two or more reactors alternately, wherein the biological sample undergoes the at least one treatment in the at least one reactor within a time period not exceeding a predetermined time period; and
   discharging the treated biological sample from the reactor array to the temperature regulation assembly, and changing the temperature of the treated biological sample to a second predetermined temperature using the temperature regulation assembly.
108. A non-volatile memory storing computer executable instructions, the computer executable instructions comprising:
   a module instructing to change the temperature of a biological sample to a first predetermined temperature using a temperature regulation assembly;
   a module instructing to allow the biological sample to flow from the temperature regulation assembly to a reactor array, the reactor array comprising two or more reactors, each of the reactors having an input port configured to receive the biological sample and an output port configured to discharge the treated biological sample;
   a module instructing to perform at least one treatment on the biological sample in the two or more reactors alternately, wherein the biological sample undergoes the at least one treatment in the at least one reactor within a time period not exceeding a predetermined time period; and
   a module instructing to discharge the treated biological sample from the reactor array to the temperature regulation assembly, and change the temperature of the treated biological sample to a second predetermined temperature using the temperature regulation assembly.

Although preferred embodiments of the present disclosure have been shown and described herein, it is obvious to those skilled in the art that these embodiments are provided in an exemplary way only. Numerous changes, alterations and substitutions will now occur to those skilled in the art without departing from the present disclosure. It should be understood that various alternatives to the embodiments of the present disclosure described herein may be employed in practicing the present disclosure. It is intended that the following claims define the scope of the present disclosure and that methods and structures within the scope of these claims and their equivalents be covered thereby.

## Claims

1. A system for treating a biological sample, the system comprising:
a temperature regulation assembly configured to change the temperature of a biological sample flowing therethrough; and
a reactor array coupled to the temperature regulation assembly, the reactor array comprising two or more reactors, each reactor in the reactor array being configured to cause the biological sample to undergo at least one treatment, each of the reactors having an input port configured to receive the biological sample and an output port configured to discharge the treated biological sample,
wherein the treatment is performed in the two or more reactors alternately, such that the time interval between completion of discharge of the treated biological sample from a first reactor and start of discharge of the treated biological sample from a second reactor is less than a predetermined time period, and the second reactor is immediately next to the first reactor in the reactor array.

2. The system according to claim 1, wherein the predetermined time period is less than five minutes.

3. The system according to claim 1, wherein the volume of each reactor in the reactor array does not exceed 5 L.

4. The system according to claim 1, wherein the temperature regulation assembly comprises a first temperature regulation device, and the first temperature regulation device is configured to increase the temperature of the biological sample to a first predetermined temperature within a first time period.

5. The system according to claim 1, wherein the biological sample comprises nucleic acid.

6. The system according to claim 5, wherein the at least one treatment comprises nucleic acid denaturation.

7. The system according to claim 1, further comprising a flushing device configured to flush the interior of at least one reactor in the reactor array.

8. A method for treating a biological sample, the method comprising:
changing the temperature of a biological sample to a first predetermined temperature using a temperature regulation assembly;
allowing the biological sample to flow from the temperature regulation assembly to a reactor array, the reactor array comprising two or more reactors, each of the reactors having an input port configured to receive the biological sample and an output port configured to discharge the treated biological sample;
causing the biological sample to undergo at least one treatment in at least one reactor in the reactor array; and
discharging the treated biological sample from the reactor array to the temperature regulation assembly, and changing the temperature of the treated biological sample to a second predetermined temperature using the temperature regulation assembly,
wherein the treatment is performed in the two or more reactors alternately, such that the time interval between completion of discharge of the treated biological sample from a first reactor and start of discharge of the treated biological sample from a second reactor is less than a predetermined time period, and the second reactor is immediately next to the first reactor in the reactor array.

9. A system for treating a biological sample, the system comprising:
a temperature regulation assembly configured to change the temperature of a biological sample flowing therethrough; and
a reactor array coupled to the temperature regulation assembly, the reactor array comprising two or more reactors, each reactor in the reactor array being configured to cause the biological sample to undergo at least one treatment, each of the reactors having an input port configured to receive the biological sample and an output port configured to discharge the treated biological sample,
wherein the treatment in the two or more reactors is coordinated so that the time interval between completion of the treatment in a first reactor and completion of the treatment in a second reactor is less than a predetermined time period, and the second reactor is immediately next to the first reactor in the reactor array.

10. A method for treating a biological sample, the method comprising:
changing the temperature of a biological sample to a first predetermined temperature using a temperature regulation assembly;
allowing the biological sample to flow from the temperature regulation assembly to a reactor array, the reactor array comprising two or more reactors, each of the reactors having an input port configured to receive the biological sample and an output port configured to discharge the treated biological sample;
causing the biological sample to undergo at least one treatment in at least one reactor in the reactor array; and
discharging the treated biological sample from the reactor array to the temperature regulation assembly, and changing the temperature of the treated biological sample to a second predetermined temperature using the temperature regulation assembly,
wherein the treatment in the two or more reactors is coordinated so that the time interval between completion of the treatment in a first reactor and completion of the treatment in a second reactor is less than a predetermined time period, and the second reactor is immediately next to the first reactor in the reactor array.
